# EUROPÄISCHE PATENTANMELDUNG

(11) **EP 4 467 656 A1**
(43) Veröffentlichungstag der Anmeldung: **27.11.2024**
(21) Anmeldenummer: 23174558.9
(22) Anmeldetag: 22.05.2023
(51) Int. Cl.: C12Q 1/22, A61L 2/28

(54) **VERFAHREN UND SYSTEM ZUM TESTEN EINER DEKONTAMINATION UND DEREN VERWENDUNGEN**

(71) Anmelder: ORTNER REINRAUMTECHNIK GMBH, 9500 Villach (AT)
(72) Erfinder: LENGGER, Sabine, 9545 Radenthein (AT); TORTSCHANOFF, Andreas, 9500 Villach (AT); CONSANI, Cristina, 9500 Villach (AT); AUBÖCK, Gerald, 9500 Villach (AT); KOFLER, Wolfram, 9871 Seeboden (AT)
(74) Vertreter: Dilg, Haeusler, Schindelmann Patentanwaltsgesellschaft mbH

(57) **Zusammenfassung**

Die vorliegende Erfindung betrifft ein Verfahren zum Testen einer Dekontamination, wobei das Verfahren ein Bereitstellen mindestens eines Filters, der Sporen aufweist, ein Unterziehen des Filters einer Dekontamination, ein Waschen des Filters, ein Inkontaktbringen des Filters mit einem Keiminduktor und ein Inkubieren des Filters und ein Bestimmen der von den Sporen bei der Keimung freigesetzten Dipicolinsäure umfasst. Des Weiteren betrifft die vorliegende Erfindung ein System zum Testen der Wirksamkeit einer Dekontamination bzw. einer Sterilisierung sowie Verwendungen des Verfahrens und des Systems.

## Beschreibung

### GEBIET DER ERFINDUNG

Die vorliegende Erfindung betrifft ein Verfahren und ein System zum Testen bzw. Überprüfen der Wirksamkeit einer Dekontamination bzw. einer Desinfektion, insbesondere einer Sterilisierung. Die vorliegende Erfindung betrifft ferner Verwendungen des Verfahrens und des Systems.

### HINTERGRUND UND STAND DER TECHNIK

Bakterielle Endosporen (wie *Clostridium perfringens*) sind allgegenwärtig und wichtige Indikatoren für die Wasserqualität und insbesondere für die Behandlungseffizienz. Besonders resistente Endosporen eines bestimmten Organismus, z.B. *Geobacillus stearothermophilus,* werden verwendet, um die Effizienz von Sterilisations- und Dekontaminationsprozessen zu gewährleisten.

Die Aufzählung der verbleibenden lebensfähigen Sporen nach der Sterilisation erfolgt routinemäßig durch Kultivierungsmethoden wie MPN-Verfahren (*Most Probable Number*) oder Filterung, gefolgt von Agar-Inkubation und Koloniezählung, die alle Fähigkeiten erfordern, um sie durchzuführen und Ergebnisse nur mit einer Zeitverzögerung liefern. Die aktuelle Technik umfasst die Zugabe von endosporbeschichteten Metallplättchen zu einer Sterilisations- oder Dekontaminationsumgebung. Die Überlebensraten dieser Endosporen werden dann durch Kultivieren und Zählen ermittelt, eine manuelle Technik, die gut etabliert ist und von verschiedenen ISO-Normen geregelt wird, mit den offensichtlichen Mängeln der manuellen Arbeitsanforderungen und der Zeit bis zum Ergebnis von bis zu 7 Tagen. Molekulare Methoden wie die PCR (*polymerase chain reaction*) sind schneller, eine Unterscheidung zwischen lebensfähigen und nicht lebensfähigen Sporen ist jedoch nicht möglich.

Ein vielversprechender Ansatz stellt die Verwendung von Dipicolinsäure (DPA) dar. Endosporen setzen DPA bei Zerstörung (alle Endosporen) und Keimung (lebensfähige Endosporen) frei, ein Umstand der auch verwendet werden kann, um ihr Vorhandensein oder ihre Häufigkeit in Umweltproben oder nach der Sterilisation abzuschätzen. Diese Methode ist viel schneller als die Kultivierung und liefert einen Hinweis auf lebensfähige Sporen innerhalb von weniger als 30 Minuten. DPA kann durch Komplexierung mit Lanthaniden (z.B., Tb³⁺) oder auf Lanthaniden basierenden Metallkomplexen nachgewiesen und quantifiziert werden. Andere in der Matrix vorhandene Verbindungen (einschließlich DPA, welche während der Sterilisation freigesetzt wird) können jedoch einen falsch positiven Effekt erzeugen, so dass nicht nur keimungsbedingte DPA (d.h. von lebensfähigen Sporen) gemessen wird.

Die DPA-basierte Technik ist aber nach derzeitigem Wissen der Erfinder nicht in Gebrauch. Zwei US-Patente wurden im Zusammenhang mit DPA in Sporen eingereicht: US 6,599,715 B1 beschreibt die Anregung der Keimung, gefolgt von einer DPA-Messung, und US 7,713,914 B2 beschreibt die Freisetzung einer Chemikalie aus Sporen durch eine schwache Säure (wie eine Aminosäure). US 5,876,960 offenbart den Nachweis von DPA über die Fluoreszenzemission des Chelats mit Lanthanoid-Ionen wie Terbium, Tb³⁺. In US 2003/0138876 A1 wird beschrieben, wie bei dieser Technik die Fluoreszenz eines Tb³⁺-DPA-Komplexes mit einem spezifischen Liganden erhöht werden kann, um die Nachweisgrenze zu verringern. In der EP 1 443 973 B1 wird vorgeschlagen, die Wirksamkeit der Sterilisation durch Sporenindikatoren und DPA zu bewerten. Bei den bisherigen DPA-basierten Techniken ist aber nach wie vor eine zuverlässige Unterscheidung zwischen abgetöteten und lebensfähigen Sporen nicht möglich, so dass deren Einsatz für die Überprüfung der Wirksamkeit einer Dekontamination stark eingeschränkt ist.

### AUFGABE DER ERFINDUNG

Eine Aufgabe der vorliegenden Erfindung ist es daher, ein verbessertes Verfahren und System zur Überprüfung der Wirksamkeit einer Dekontamination bereitzustellen, das schneller (insbesondere im Vergleich zu herkömmlichen Kultivierungsmethoden) und zuverlässiger (insbesondere im Vergleich zu bisherigen Verfahren auf Basis der Bestimmung von DPA, ganz besonders im Hinblick darauf, dass nur die lebensfähigen Sporen - also diejenigen Sporen, bei denen die Dekontamination nicht erfolgreich war - bestimmt werden) Ergebnisse zur Wirksamkeit bzw. Effizienz einer Dekontamination liefert und automatisierbare und miniaturisierbare Lösungen und damit auch hohe Durchsatzgeschwindigkeiten und Mehrkanalsysteme ermöglicht.

### ZUSAMMENFASSUNG DER ERFINDUNG

Die Erfinder der vorliegenden Erfindung haben umfangreiche Untersuchungen durchgeführt und haben dabei herausgefunden, dass durch Immobilisieren von Sporen auf einem Filter, der anschließend einem zu überprüfenden Dekontaminationsvorgang ausgesetzt wird, die durch Absterben von Sporen bei der Dekontamination freigesetzte DPA vor dem Bestimmen der (noch) lebensfähigen Sporen durch Waschen des Filters in einfacher und automatisierbarer Weise entfernt werden kann, so dass bei der anschließenden Bestimmung mittels Keimung der lebensfähigen Sporen im Wesentlichen nur die bei der Keimung freigesetzte DPA erfasst wird und somit eine zuverlässige Aussage getroffen werden kann, ob und inwieweit die Dekontamination wirksam bzw. erfolgreich war.

Die vorliegende Erfindung betrifft dementsprechend ein Verfahren zum Testen bzw. Überprüfen einer Dekontamination bzw. einer Sterilisierung, wobei das Verfahren ein Bereitstellen mindestens eines Filters, der (insbesondere auf einer Oberfläche) Sporen (insbesondere bakterielle Endosporen) aufweist, ein Unterziehen (Aussetzen, Unterwerfen) des Filters einer Dekontamination (einem Dekontaminationsvorgang, einem Dekontaminationsprozess), ein Waschen des Filters (zur Entfernung von (bei der Dekontamination freigesetzter) Dipicolinsäure (DPA) und/oder störender Komponenten, wobei die Sporen aber zurückgehalten werden), ein Inkontaktbringen des Filters mit einem Keiminduktor und ein Inkubieren des Filters (zum Induzieren der Keimung der (nach der Dekontamination noch lebensfähigen bzw. keimfähigen) Sporen) und ein Bestimmen der von den Sporen bei der Keimung freigesetzten DPA umfasst.

Des Weiteren betrifft die vorliegende Erfindung ein System (Anordnung, Vorrichtung) zum Testen bzw. Überprüfen einer Dekontamination bzw. einer Sterilisierung, insbesondere zum Durchführen eines Verfahrens wie hierin beschrieben, wobei das System mindestens einen Filter, der (insbesondere auf einer Oberfläche) Sporen (insbesondere bakterielle Endosporen) aufweist, eine Dekontaminationseinheit (insbesondere eine Einheit, die konfiguriert ist, den Filter einer Dekontamination zu unterziehen); eine Einheit (Wascheinheit), die konfiguriert ist, den Filter zu waschen, eine Einheit (Kontakt- und Inkubationseinheit), die konfiguriert ist, den Filter mit einem Keiminduktor in Kontakt zu bringen und zu inkubieren, und eine Einheit (Bestimmungseinheit), die konfiguriert ist, die von den Sporen bei der Keimung freigesetzte DPA zu bestimmen, aufweist.

Ferner betrifft die vorliegende Erfindung die Verwendung eines Verfahrens wie hierin beschrieben oder eines Systems wie hierin beschrieben zum Bestimmen der Wirksamkeit (Effizienz) einer Dekontamination (eines Dekontaminationsvorgangs, eines Dekontaminationsprozesses).

Weitere Aufgaben und Vorteile von Ausführungsformen der vorliegenden Erfindung werden an Hand der folgenden detaillierten Beschreibung und der beigefügten Abbildung ersichtlich.

### KURZE BESCHREIBUNG DER ABBILDUNG

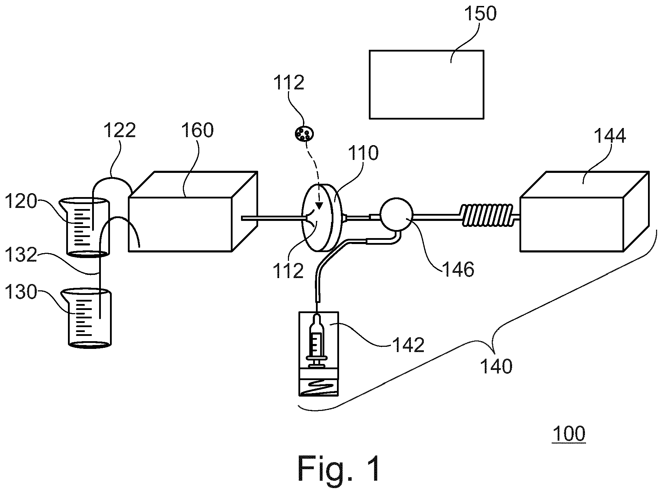

Abbildung 1 zeigt ein System zum Testen einer Dekontamination gemäß einer beispielhaften Ausführungsform, das zum Durchführen eines Verfahrens gemäß einer beispielhaften Ausführungsform geeignet ist.

### DETAILLIERTE BESCHREIBUNG DER ERFINDUNG

Im Folgenden werden nähere Details der vorliegenden Erfindung und weitere Ausführungsformen davon beschrieben. Die vorliegende Erfindung ist jedoch nicht auf die folgende detaillierte Beschreibung beschränkt, sondern sie dient lediglich der Veranschaulichung der erfindungsgemäßen Lehren.

Es sei darauf hingewiesen, dass Merkmale, die im Zusammenhang mit einer beispielhaften Ausführungsform beschrieben werden, mit jeder anderen beispielhaften Ausführungsform kombiniert werden können. Insbesondere können Merkmale, die im Zusammenhang mit einer beispielhaften Ausführungsform eines erfindungsgemäßen Verfahrens beschrieben werden, mit jeder anderen beispielhaften Ausführungsform eines erfindungsgemäßen Verfahrens sowie mit jeder beispielhaften Ausführungsform eines erfindungsgemäßen Systems sowie jeder beispielhaften Ausführungsform einer erfindungsgemäßen Verwendung kombiniert werden und umgekehrt, sofern nicht ausdrücklich etwas anderes vermerkt ist.

Wenn ein Begriff mit einem unbestimmten oder bestimmten Artikel, wie zum Beispiel "ein", "eine", "eines", "der", "die" und "das", im Singular bezeichnet wird, schließt dies auch den Begriff im Plural mit ein und umgekehrt, sofern der Kontext nicht eindeutig anderes festlegt. Die Ausdrücke "aufweisen" bzw. "umfassen", wie sie hier verwendet werden, schließen nicht nur die Bedeutung von "enthalten" oder "beinhalten" ein, sondern können auch "bestehen aus" und "im Wesentlichen bestehen aus" bedeuten.

In einem ersten Aspekt betrifft die vorliegende Erfindung ein Verfahren zum Testen bzw. Überprüfen der Wirksamkeit einer Dekontamination bzw. einer Sterilisierung.

Unter dem Begriff "Dekontamination" wird im Rahmen der vorliegenden Anmeldung insbesondere ein Entfernen von mikrobiellen Verunreinigungen verstanden, im Unterschied zur Entfernung von radioaktiven oder sonstigen toxischen chemischen Verunreinigungen. Mikrobielle Verunreinigungen betreffen Mikroorganismen, wie insbesondere Bakterien, Viren, Hefen und Pilze, einschließlich ihrer Ruhestadien wie Sporen. Bei der Dekontamination sollen diese Mikroorganismen weitestgehend abgetötet oder inaktiviert werden. Dementsprechend kann im Rahmen der vorliegenden Anmeldung die Dekontamination auch als eine Desinfizierung bzw. eine Desinfektion bezeichnet werden, worunter insbesondere eine Reduzierung der Zahl an Infektionserreger auf einer Fläche oder einem Gegenstand, so dass davon keine Infektion ausgehen kann, verstanden wird. Gemäß einer Ausführungsform kann die Dekontamination auch eine Sterilisierung bzw. eine Sterilisation umfassen, worunter insbesondere eine Behandlung eines Gegenstandes, so dass er frei von vermehrungsfähigen Mikroorganismen ist, verstanden wird.

Gemäß einer beispielhaften Ausführungsform wird in dem Verfahren zunächst ein Filter bereitgestellt, der Sporen aufweist. Insbesondere können sich die Sporen auf einer Oberfläche des Filters befinden. Bei den Sporen kann es sich insbesondere um bakterielle Endosporen handeln, wie zum Beispiel um Endosporen von *Clostridium perfringens* oder *Geobacillus stearothermophilus.* Letztere sind aufgrund ihrer ausgeprägten Resistenz besonders geeignet für das Überprüfen der Wirksamkeit einer Dekontamination. Zur Erleichterung der späteren Auswertung, insbesondere falls nicht nur eine qualitative oder semiquantitative, sondern auch eine quantitative Aussage gewünscht ist, ist es vorteilhaft, wenn die Anzahl an Sporen auf dem Filter bekannt ist.

Gemäß einer beispielhaften Ausführungsform umfasst das Bereitstellen des Filters ein Ablagern von Sporen auf dem Filter, zum Beispiel durch Leiten einer sporenhaltigen Flüssigkeit durch den Filter, auf dem die Sporen zurückgehalten werden. Bei bekannter Menge an Sporen in der Flüssigkeit, was bei einer kommerziell erhältlichen Sporenlösung regelmäßig der Fall ist, kann hierdurch in besonders einfacher und effizienter Weise ein mit Sporen beladener Filter mit bekannter Anzahl an Sporen auf dem Filter erhalten werden. Der Filter kann aber auch auf andere Weise mit Sporen versehen werden.

Gemäß einer beispielhaften Ausführungsform können in dem Verfahren auch mehrere Filter eingesetzt werden, insbesondere mindestens 2, 3, 4, 5, 10, 20 oder mehr Filter. In vorteilhafter Weise können mehrere Filter parallel zueinander angeordnet sein, wodurch insbesondere mehrere Tests zur Überprüfung einer Dekontamination gleichzeitig durchgeführt werden können und somit ein besonders hoher Durchsatz erreicht werden kann.

Gemäß einer beispielhaften Ausführungsform ist der Filter ausgewählt aus der Gruppe, bestehend aus einem Membranfilter, einem Oberflächenfilter und einem Tiefenfilter.

Gemäß einer beispielhaften Ausführungsform ist der Filter aus Polytetrafluorethylen (PTFE) und/oder Polyvinylidenfluorid (PVDF) und/oder Glasfaser und/oder Nylon gemacht. Diese Materialien haben sich als besonders geeignet für das erfindungsgemäße Verfahren erwiesen. Der Filter kann aber auch aus anderen Materialien gemacht sein, solange er in der Lage ist Sporen zurückzuhalten und der zu überprüfenden Dekontaminationsbehandlung standhält.

Gemäß einer beispielhaften Ausführungsform hat der Filter eine Porengröße zwischen 0,1 und 1 µm, insbesondere von 0,2 bis 0,5 µm, insbesondere von 0,25 bis 0,4 µm. Ein Filter mit dieser Porengrößen kann Sporen quantitativ zurückhalten, sich dennoch aber problemlos (insbesondere ohne übermäßigen Druckaufbau) Durchspülen lassen. Die Auswahl einer geeigneten Porengröße kann von einem Fachmann auf Basis der erwarteten Menge und Größe der Sporen getroffen werden. Im Rahmen der vorliegenden Anmeldung kann unter dem Begriff "Porengröße" insbesondere die nominale Porengröße verstanden werden, die das Maximum in einer Porengrößenverteilung wiedergibt und nach üblichen, einem Fachmann bekannten porosimetrischen Methoden bestimmt werden kann.

Gemäß einer beispielhaften Ausführungsform kann das Verfahren ferner ein (erstes) Waschen des Filters vor dem Unterziehen des Filters einer Dekontamination umfassen. Dieser optionale Waschschritt dient zur Entfernung von extrazellulärem DPA, welches gegebenenfalls ein starkes Hintergrundsignal erzeugen kann. Das Waschen kann mit einer Waschlösung, z.B. einer Pufferlösung, erfolgen, die zum Beispiel 10 mM Tris(hydroxymethyl)aminomethan (Tris) in Wasser bei pH 8 enthalten kann. Die Waschlösung kann beispielsweise automatisiert unter Verwendung einer Spritzenpumpe oder Mikropumpe bei einer Flussrate von 1 bis 4 ml/min für 1 bis 4 Minuten durch den Filter gespült werden bzw. bei einem Einsatz von mehreren Filtern in paralleler Anordnung eine entsprechend angepasste Flussrate.

Gemäß einer beispielhaften Ausführungsform wird in dem Verfahren der Filter einer Dekontamination (einem Dekontaminationsvorgang, einem Dekontaminationsprozess) unterzogen (ausgesetzt, unterworfen). Hierzu kann der Filter in eine Dekontaminationsvorrichtung gegeben werden, in der die auf ihre Wirksamkeit zu testende Dekontamination durchgeführt wird.

Gemäß einer beispielhaften Ausführungsform umfasst das Unterziehen des Filters einer Dekontamination einen Dekontaminationsvorgang, der aus der Gruppe, bestehend aus einer Heiß-Dampf-Sterilisation (Autoklavieren), einem Dekontaminationsprozess mit H₂O₂ (insbesondere VHP-Technik, d.h. mit verdampftem Wasserstoffperoxid), einem Reinigungs-Entkeimungsprozess in der Abwassertechnik, ausgewählt ist.

Gemäß einer beispielhaften Ausführungsform wird in dem Verfahren der Filter nach der Dekontamination gewaschen. Hierdurch kann bei der Dekontamination freigesetzte DPA (d.h. DPA, die beim Absterben von Sporen während der Dekontamination freigesetzt wird) und/oder andere störende Komponenten von dem Filter entfernt werden, wobei die Sporen aber zurückgehalten werden. Zu diesem Zweck kann der Filter mit einer Waschlösung, insbesondere einer Pufferlösung, durchströmt werden, die wie beim oben diskutierten optionalen (ersten) Waschen zum Beispiel 10 mM Tris(hydroxymethyl)aminomethan (Tris) in Wasser bei pH 8 enthalten kann. Die Waschlösung kann beispielsweise automatisiert unter Verwendung einer Spritzenpumpe oder Mikropumpe bei einer Flussrate von 1 bis 4 ml/min für 1 bis 4 Minuten durch den Filter gespült werden. Das Waschen des Filters zur Entfernung von bei der Dekontamination freigesetzter DPA kann hierin auch als zweites Waschen bezeichnet werden, wobei noch einmal klargestellt wird, dass das oben diskutierte erste Waschen nicht essenziell ist.

Gemäß einer beispielhaften Ausführungsform kann das Verfahren nach dem (zweiten) Waschen des Filters ferner ein Erwärmen des Filters und/oder einer Waschflüssigkeit und anschließend ein (drittes) Waschen des Filters mit der (erwärmten) Waschflüssigkeit umfassen. Hierbei kann der Filter und/oder die Waschflüssigkeit zum Beispiel auf 60 bis 70 °C erwärmt werden. Dieser optionale "warme" Waschschritt dient zur bestmöglichen Entfernung von Material von Sporen, die zwar abgetötet aber deren Membrane nicht vollständig zerstört wurden. Das Waschen kann abermals mit einer Waschlösung, z.B. einer Pufferlösung, erfolgen, die zum Beispiel 10 mM Tris(hydroxymethyl)aminomethan (Tris) in Wasser bei pH 8 enthalten kann. Die Waschlösung kann zusätzlich auch einen Keiminduktor (z.B. L-Valin) enthalten, wobei in diesem Fall das dritte Waschen möglichst kurz bzw. bei einer möglichst niedrigen Temperatur durchgeführt wird, um ein vorzeitiges Keimen der Sporen zu vermeiden. Die Waschlösung kann beispielsweise bei einer Flussrate von 1 bis 4 ml/min für 2 bis 5 Minuten durch den Filter gespült werden.

Gemäß einer beispielhaften Ausführungsform wird in dem Verfahren der Filter mit einem Keiminduktor in Kontakt gebracht und inkubiert. Unter einem "Keiminduktor" wird im Rahmen der vorliegenden Anmeldung insbesondere ein Mittel verstanden, das ein Keimen von Sporen auslösen kann; mit anderen Worten ein Mittel zur Keimung der Sporen. Bei der Keimung setzen die nach der Dekontamination noch lebensfähigen Sporen DPA frei, die anschließend (insbesondere quantitativ) bestimmt werden kann und somit ein Überprüfen der Wirksamkeit einer Dekontamination ermöglicht.

Gemäß einer beispielhaften Ausführungsform umfasst das Inkontaktbringen und Inkubieren des Filters mit einem Keiminduktor eine Zugabe des Keiminduktors zu dem Filter für eine bestimmte Zeit, insbesondere für eine Zeit, die für ein Keimen weitestgehend sämtlicher noch lebensfähiger Sporen ausreicht. Je nach verwendeter Art an Sporen und Temperatur kann eine hierfür ausreichende Zeit für einen Fachmann anhand seines Fachwissens in geeigneter Weise gewählt werden. Typische Inkubationszeiten liegen im Bereich von 15 bis 60 Minuten, zum Beispiel 30 bis 45 Minuten. Es kann aber auch schon eine Inkubationszeit von 5 oder 10 Minuten ausreichend sein. Das Inkontaktbringen des Filters mit einem Keiminduktor kann beispielsweise ein Durchleiten einer Lösung, die den Keiminduktor enthält, durch den Filter für eine bestimmte Zeit umfassen. Zusätzlich kann der Filter und/oder die Lösung, die den Keiminduktor enthält, beim Inkubieren auf eine dem Wachstum förderliche Temperatur erwärmt werden, zum Beispiel auf eine Temperatur im Bereich von 57 und 70°C, und bei der erhöhten Temperatur über die Inkubationszeit gehalten werden.

Gemäß einer beispielhaften Ausführungsform ist der Keiminduktor ausgewählt aus der Gruppe, bestehend aus Valin, insbesondere L-Valin, L-Alanin oder eine Mischung aus L-Asparagin, D-Glucose, D-Fructose und Kalium-Ionen (AGFK). Diese haben sich als geeignete Keiminduktoren erwiesen, die zudem die anschließende Bestimmung der von den Sporen bei der Keimung freigesetzten DPA nicht stören, im Unterschied zu beispielsweise dem Keiminduktor Dodecylamin.

Gemäß einer beispielhaften Ausführungsform enthält die Lösung, die den Keiminduktor enthält, ferner eine Puffersubstanz. Als geeignete Puffersubstanzen haben sich Tris(hydroxymethyl)aminomethan (Tris) und Acetat erwiesen, da sie die anschließende Bestimmung der von den Sporen bei der Keimung freigesetzten DPA nicht stören, im Unterschied zu beispielsweise Phosphat.

Gemäß einer beispielhaften Ausführungsform wird in dem Verfahren die von den Sporen bei der Keimung freigesetzte DPA bestimmt. Durch einen Vergleich der so bestimmten Menge an DPA, die mit der Anzahl an nach der Dekontamination noch lebensfähigen Sporen korreliert, zu der ursprünglich eingesetzten Menge an Sporen, lässt sich die Wirksamkeit der Dekontamination testen.

Gemäß einer beispielhaften Ausführungsform erfolgt das Bestimmen der von den Sporen bei der Keimung freigesetzten DPA mittels einer fluoreszenzspektroskopischen Methode.

Gemäß einer beispielhaften Ausführungsform umfasst das Bestimmen der von den Sporen bei der Keimung freigesetzten DPA ein Inkontaktbringen der bei der Keimung freigesetzten DPA mit Lanthanoid-Ionen (oder einem Lanthanoid-Komplex) und ein anschließendes Detektieren der Fluoreszenz des gebildeten Lanthanoid-DPA-Komplexes. Beispielsweise kann das Inkontaktbringen der bei der Keimung freigesetzten DPA mit Lanthanoid-Ionen ein Durchleiten einer Lösung von Lanthanoid-Ionen durch den Filter oder eine Zugabe einer Lösung von Lanthanoid-Ionen zu einer Lösung, die die bei der Keimung freigesetzte DPA enthält, umfassen. Als geeignete Lanthanoid-Ionen haben sich insbesondere Terbium-Ionen, Tb³⁺, erwiesen. Andere Lanthanoid-Ionen können aber ebenfalls verwendet werden, solange sie mit DPA einen mittels Fluoreszenz oder anderweitig detektierbaren Komplex bilden. Die Lösung von Lanthanoid-Ionen kann insbesondere Terbiumchlorid, TbCl₃, enthalten, oder ein anderes in Wasser lösliches Terbiumsalz, dessen Anion bei der anschließenden Detektion nicht stört. Die Lanthanoid-Ionen können in der Lösung von Lanthanoid-Ionen auch als ein Lanthanoid-Komplex vorliegen, wobei der Ligand vorzugsweise eine geringere Komplexbildungskonstante als DPA gegenüber den Lanthanoid-Ionen aufweist. Das Detektieren der Fluoreszenz des gebildeten Lanthanoid-DPA-Komplexes kann beispielsweise mittels eines Fluoreszenzdetektors mit einer Durchflussküvette durchgeführt werden.

Das Verfahren kann insbesondere mittels eines Systems, wie nachstehend weiter ausgeführt, durchgeführt werden.

In einem weiteren Aspekt betrifft die vorliegende Erfindung ein System (Anordnung, Vorrichtung) zum Testen bzw. Überprüfen der Wirksamkeit einer Dekontamination bzw. einer Sterilisierung. Das System kann insbesondere zum Durchführen eines Verfahrens wie hierin beschrieben geeignet sein.

Gemäß einer beispielhaften Ausführungsform weist das System einen Filter auf, der Sporen aufweist. Zu näheren Details zu dem Filter, einschließlich wie er erhalten werden kann, wird auf die detaillierten Ausführungen im Zusammenhang mit dem erfindungsgemäßen Verfahren verwiesen.

Gemäß einer beispielhaften Ausführungsform weist das System eine Dekontaminationseinheit auf, insbesondere eine Einheit, die konfiguriert ist, den Filter einer Dekontamination zu unterziehen. Zu diesem Zweck kann der Filter in die Dekontaminationseinheit gegeben werden, in der die auf ihre Wirksamkeit zu testende Dekontamination durchgeführt wird. In der Dekontaminationseinheit kann beispielsweise eine Heiß-Dampf-Sterilisation (Autoklavieren), ein Dekontaminationsprozess mit H₂O₂ (insbesondere VHP-Technik, d.h. mit verdampftem Wasserstoffperoxid)und/oder ein Reinigungs-Entkeimungsprozess in der Abwassertechnik durchgeführt werden.

Gemäß einer beispielhaften Ausführungsform weist das System eine Einheit auf, die konfiguriert ist, den Filter zu waschen. Diese Einheit kann dementsprechend auch als eine Wascheinheit bezeichnet werden. Die Wascheinheit kann insbesondere einen Vorratsbehälter für eine Waschflüssigkeit und eine Zuleitung vom Vorratsbehälter zum Filter, ggf. über eine Pumpvorrichtung, umfassen.

Gemäß einer beispielhaften Ausführungsform weist das System eine Einheit auf, die konfiguriert ist, den Filter mit einem Keiminduktor in Kontakt zu bringen und zu inkubieren. Diese Einheit kann dementsprechend auch als eine Kontakt- und Inkubationseinheit bezeichnet werden. Die Kontakt- und Inkubationseinheit kann insbesondere einen Vorratsbehälter für einen Keiminduktor, der beispielsweise in gelöster Form vorliegt, also eine Lösung, die den Keiminduktor enthält, und eine Zuleitung vom Vorratsbehälter zum Filter, ggf. über eine Pumpvorrichtung, umfassen.

Gemäß einer beispielhaften Ausführungsform weist das System eine Einheit auf, die konfiguriert ist, die von den Sporen bei der Keimung freigesetzte DPA zu bestimmen. Diese Einheit kann dementsprechend auch als eine Bestimmungseinheit bezeichnet werden. Die Bestimmungseinheit kann insbesondere eine Zugabevorrichtung für beispielsweise eine Lösung von Lanthanoid-Ionen zum Filter bzw. zu der von den Sporen bei der Keimung freigesetzten DPA und eine Detektionseinheit, beispielsweise einen Fluoreszenzdetektor umfassen.

Gemäß einer beispielhaften Ausführungsform sind zumindest der Filter, die Wascheinheit, die Kontakt- und Inkubationseinheit und die

Bestimmungseinheit fluidisch miteinander verbindbar bzw. fluidisch miteinander verbunden. Insbesondere kann es sich dabei um ein mikrofluidisches System und/oder ein Durchfluss-System handeln. Mit anderen Worten kann der Filter in eine fluidische Umgebung integriert sein.

Gemäß einer beispielhaften Ausführungsform weist das System ferner mindestens eine der folgenden Komponenten auf: eine Pumpvorrichtung zum Fördern der diversen Flüssigkeiten durch das System; ein Ventil zum Steuern der Fluidströme, insbesondere zum Gewähren bzw. Unterbrechen bestimmter Fluidströme; eine Zugabevorrichtung bzw. ein Dosiersystem, insbesondere zur dosierten Zugabe beispielsweise einer Lösung von Lanthanoid-Ionen zum Filter bzw. zu der von den Sporen bei der Keimung freigesetzten DPA, zum Beispiel über ein Spritzenpumpen-Dosiersystem; und einen (oder mehrere) Vorratsbehälter (Reservoir) zum Bereitstellen der diversen Flüssigkeiten.

Gemäß einer beispielhaften Ausführungsform ist das System ein automatisiertes System, das einen oder mehrere Vorratsbehälter (für die Lösungsmittel und Reagenzien), eine oder mehrere Pumpvorrichtungen (zum Spülen der Flüssigkeiten durch den Filter bzw. zum Einspritzen der Reagenzien (z.B. Lanthanid), gefolgt von Mischeinheiten), einen (vorzugsweise beheizten bzw. beheizbaren) Filterhalter, eine Einheit, die konfiguriert ist, die von den Sporen bei der Keimung freigesetzte Dipicolinsäure zu bestimmen (zum Beispiel eine Spektroskopie-Durchflusszelle, eine spektroskopische Einheit für die Fluoreszenzdetektion einschließlich Lichtquelle, Optik und optischem Detektor), ein Datenerfassungs- und Kontrollsystem, eine Software einschließlich einer Benutzeroberfläche umfasst.

In einem weiteren Aspekt betrifft die vorliegende Erfindung die Verwendung eines Verfahrens wie hierin beschrieben oder eines Systems wie hierin beschrieben zum Bestimmen der Wirksamkeit bzw. der Effizienz einer Dekontamination (eines Dekontaminationsvorgangs, eines Dekontaminationsprozesses). Wie vorstehend ausgeführt, lässt sich durch Vergleich der bei dem Verfahren bzw. mittels des Systems bestimmten Menge an DPA, die mit der Anzahl an nach der Dekontamination noch lebensfähigen Sporen korreliert, zu der ursprünglich eingesetzten Menge an Sporen, die Wirksamkeit der Dekontamination überprüfen.

Gemäß einer beispielhaften Ausführungsform umfasst die Dekontamination einen Dekontaminationsvorgang, der aus der Gruppe, bestehend aus einer Heiß-Dampf-Sterilisation (Autoklavieren), einem Dekontaminationsprozess mit HzOz (insbesondere VHP-Technik, d.h. mit verdampftem Wasserstoffperoxid), einem Reinigungs-Entkeimungsprozess in der Abwassertechnik, ausgewählt ist.

Die vorliegende Erfindung wird weiterhin an Hand der folgenden Beispiele beschrieben, die aber lediglich der Verdeutlichung der erfindungsgemäßen Lehren dienen und in keiner Weise den Umfang der vorliegenden Erfindung beschränken sollen.

### Beispiele

Im Folgenden werden exemplarische Versuche beschrieben, welche die erfindungsgemäße Lehre experimentell belegen.

Eine kommerziell erhältliche Sporenlösung (Merck, *Geobacillus stearothermophilus Spore Suspension,* Produkt Nr. 1.11499) mit einer Konzentration von 10⁻⁸ CFU/ml wurde verwendet. 10 µl der Lösung wurden jeweils mittig auf einen mikrobiologischen PTFE-Filter (13 mm Durchmesser, 0,2 µm Porengröße, hydrophob) aufgetragen und bei 35°C für 1 h getrocknet. Diese Filter wurden dann weiters in einen sterilen (autoklavierten) Filterhalter eingebracht, um mit Pufferlösungen gewaschen, oder zum Zwecke der Inkubation mit Pufferlösung gefüllt zu werden. Dabei wurde die Seite, auf der die Sporen aufgebracht wurden, immer gegen die Flussrichtung gerichtet, um etwaigen Verlust der Sporen zu verhindern.

Der so vorbereitete Filter wurde gewaschen, indem 4 ml einer Pufferlösung (Tris, 10mM, pH=8) durchgepumpt werden, zum Beispiel bei einer Flussrate von 1ml/min. Dadurch wird extrazelluläres DPA entfernt, welches gegebenenfalls ein starkes Hintergrundsignal erzeugen könnte. Danach wird der Filter dem Fluidsystem entnommen, dem Sterilisationsprozess ausgesetzt und danach wieder in den Filterhalter eingesetzt. Daraufhin wird der Filter gewaschen (1x mit 4ml Pufferlösung), um durch den Sterilisationsprozess aus den Zellen freigesetztes DPA zu entfernen. Zur Sicherheit werden die Proben 5 Minuten auf 65°C erhitzt und danach 2x mit Pufferlösung gewaschen, welcher bereits L-Valin (1mM) zugesetzt wurde. Dieser Schritt dient dazu, Material von Sporen, die zwar abgetötet aber deren Membrane nicht vollständig zerstört wurden, möglichst gut zu entfernen. Hier ist zu beachten, dass keinesfalls länger als 5 min und möglichst in reinem Puffer (noch ohne L-Valin) erhitzt wird, da gezeigt wurde, dass es unter diesen Bedingungen in diesem Zeitraum noch zu keiner nennenswerter Keimung kommt (T. Zhou et al., PloS one, Vol. 8, Nr. 9, S. e74987, 2016). Dieser Schritt kann bei der Verwendung von anderen Sporen gegebenenfalls angepasst werden, und eventuell auch ganz entfallen. Danach wird die Probe bei 65°C für 30 Minten inkubiert in Pufferlösung mit L-Valin und danach der Filter mit 2ml derselben Lösung (Puffer mit L-Valin) ausgespült. Um aktive Sporen nachzuweisen, wird diese Lösung nun auf ihren DPA-Gehalt untersucht.

Alternativ könnte das Experiment auch mit anderen Sporen und anderen Pufferlösungen durchgeführt werden. Die Pufferlösung muss so beschaffen sein, dass sie nicht mit der DPA-Quantifizierung, welche typischerweise auf Fluoreszenzmessungen basiert, interferiert (z.B. durch Unterdrücken der Fluoreszenz oder durch zu starke Eigenfluoreszenz) und gleichzeitig die Sporenkeimung unterstützt. In Vorversuchen zeigte sich z.B. Acetat-Puffer als ebenfalls geeignet, wohingegen Phosphat-Puffer ungeeignet ist, da die Fluoreszenz des Tb-DPA Komplexes stark unterdrückt wurde. In den Versuchen zeigte sich, dass hydrophobe Filter in Kombination mit den verwendeten Sporen ein besseres Verhalten gegenüber dem gewählten Sterilisationsprozess hatten. Für andere Sterilisationsprozesse oder andere Sporen könnten aber auch andere Filter vorteilhaft sein. Typische Porengrössen sind z.B. 220nm oder 450nm.

Ebenso kann das Nährmedium und auch die Bedingungen bei der Keimung variiert werden. In der Literatur (D. E. Cortezzo et al., Journal of Applied Microbiology, 96(4) (2004) 725-741) sind alternativ zu L-Valin auch andere Keiminduktoren beschrieben, z.B. Dodecylamin, L-Alanin oder eine Mischung aus L-Asparagin, D-Glucose, D-Fructose und Kalium-Ionen (AGFK). Allerdings gelten für das Nährmedium ähnliche Voraussetzungen wie für die Pufferlösung und z.B. zeigte sich, dass Dodecylamin, welches in der Literatur als sehr effektives Nährmedium für die Keimung von *Geobacillus stearothermophilus* beschrieben wurde, ungeeignet ist, da die Fluoreszenz unterdrückt wird. Auch die genauen Bedingungen und Abfolge bei Waschprozessen und bei der Keimung können gegebenenfalls modifiziert werden. Beispielsweise ist bekannt, dass Temperatursprünge (z.B. Erhitzen für kurze Zeit auf 100°C) bei manchen Sporen geeignet sind, den Keimprozess noch zu beschleunigen (D. E. Cortezzo et al., Journal of Applied Microbiology, 96(4) (2004) 725-741).

In einer exemplarischen Versuchsreihe wurden die intermediären Waschlösungen aufbehalten und auf DPA-Gehalt analysiert.

Die DPA-Messung selbst erfolgte dabei in einem Versuchsaufbau im optischen Labor mittels zeitaufgelöster Fluoreszenz. Die Messung erfolgte in einer 0,5 ml Quarz-Küvette. 400 µl der Probe werden in der Küvette mit 40 µl Tb³⁺ Lösung (Konzentration = 10 µM) versetzt, so dass in der Probe eine Tb³⁺ Konzentration von ca. 1 µM eingestellt ist. Gegebenenfalls könnten an dieser Stelle auch Reagenzien beigesetzt werden, um die Bedingungen für die Fluoreszenzmessung zu optimieren (z.B. Einstellen des pH-Wertes). Die Probe wird mit Laserpulsen mit 266 nm und einer Repetitionsrate von 18 Hz angeregt und die emittierte Fluoreszenz wird durch eine Linse gesammelt und mittels eines kommerziellen Detektors (Ketek, PM3315-WB) gemessen. Gemessen wird dabei der zeitaufgelöste Fluoreszenz-Abfall. Am Anfang jeder Messreihe wird dabei das Signal mit DPA-Lösungen bekannter Konzentration kalibriert.

Auch alternative Möglichkeiten zum quantitativen Nachweis von DPA könnten zum Einsatz kommen. Zeitaufgelöste Fluoreszenz nach Komplexierung mit Tb³⁺ hat sich als vorteilhaft erwiesen, durch ihre hohe Empfindlichkeit und die Möglichkeit, durch das Setzen zeitlicher Fenster, Hintergrundfluoreszenz und Streulicht-Anteile auszublenden. Das Verfahren könnte z.B. durch Einsatz weiterer Optik oder anderer Komplexbildner modifiziert werden.

Folgende Proben wurden beispielhaft untersucht:
- 3 x Proben ohne Sporen, "Blank" (A, B, C)
- 3 x Proben mit Sporen und mit Sterilisation mittels gasförmigem HzOz (D, E, F), wobei Probe "D" nicht steril gehandhabt wurde, um sie der Kontamination durch Sporen aus der Umgebung auszusetzen.
- 3 x Proben mit Sporen und mit Sterilisation im Autoklaven (G, H, I)
- 1 x Referenz ohne Sterilisationsprozess (J)

Folgende Probennahmen wurden durchgeführt:
- Waschen 1: Spülen der Filter mit Pufferlösung nach Aufbringen der Sporen
- Waschen 2: erste Spülung des Filter mit Pufferlösung nach der Sterilisation
- Waschen 3: Spülung des Filters mit L-Valin Puffer nach Erhitzen
- Inkubiert 1: Spülen des Filters nach Inkubation

Die Kalibration erfolgte durch Messen von Lösungen mit bekannter DPA-Konzentration mit 0, 20, 100, und 500 nM. Eine Kenngrösse wird aus dem gemessenen Signal des Detektors abgeleitet z.B. durch Integration der Fluoreszenz-Abklingkurven über einen gewählten Zeitbereich (z.B. von 100-3500 µs).

Die Ergebnisse sind in nachstehender Tabelle 1 zusammengefasst.

**Table 1: Gemessene Konzentrationen in nM. (Negative Konzentrationen entsprechen der Messungenauigkeit)**

| **PROBE** | **WASCHEN 1** | **WASCHEN 2** | **WASCHEN 3** | **INKUBIERT 1** |
|---|---|---|---|---|
| **A** | -4 | -6 | -9 | -4 |
| **B** | -5 | -8 | -7 | -3 |
| **C** | -6 | 8 | -7 | -6 |
| **D** | 324 | 80 | 24 | 57 |
| **E** | 352 | 17 | 6 | 4 |
| **F** | 319 | 121 | -5 | -9 |
| **G** | 343 | 31 | -2 | -8 |
| **H** | 245 | - | -1 | -7 |
| **I** | 321 | 150 | -2 | -9 |
| **J** | 353 | 20 | 46 | 147 |

Die Proben A, B und C enthalten keine Sporen und dienen zur Kontrolle auf mögliche Kontaminationen und zum Abschätzen der Nachweisgrenzen und der erzielbaren Genauigkeit. Tatsächlich ist in allen Waschlösungen das gemessene Signal gleich Null.

Bei allen anderen Lösungen wurden entsprechend der Beschreibung die Filter mit Sporen beladen. In diesen Proben findet man dementsprechend nach der ersten Probenahme ("Waschen 1") eine hohe Menge an DPA. Hier handelt es sich um DPA, welches in der Sporensuspension frei enthalten ist. In "Waschen 2" und "Waschen 3" nach der Sterilisation, findet man DPA, welches während der Sterilisation durch Zerstören der Sporen freigesetzt wurde. Hier sieht man große Variationen, je nach Sterilisationsprozess und Probe. Wichtig für den Nachweis der Sterilisation ist das Ergebnis nach Inkubation ("Inkubiert 1"). Hier sieht man, dass keimfähige Sporen nur noch in den nicht-sterilen Proben ("D" und "J") vorhanden sind, was durch die Freisetzung von DPA nachgewiesen wird.

Abschließend sei darauf hingewiesen, dass diese Studie noch mit relativ hohen Sporenkonzentrationen und großen Probenvolumina durchgeführt wurde, um die prinzipielle Idee zu validieren. Während damit Sporenkonzentrationen quantifiziert werden können, ist natürlich ein wichtiges Ziel der Nachweis einzelner Sporen. Mit dem derzeitigen Set-Up kann man Nachweisgrenzen für DPA von <10 nM erzielen. Durch Anpassung der Messparameter können auch niedrigere Nachweisgrenzen erzielt werden (bis hinunter zu 10 pM), jedoch reduziert sich der dynamische Bereich und höhere Konzentrationen könnten nicht mehr quantifiziert werden. Wenn man annimmt, dass eine einzelne Spore typischerweise 2,3×10⁸ DPA-Moleküle enthält und davon nur ein gewisser Anteil freigesetzt wird, abhängig von Sporenart und Umgebungsparametern. In der Literatur werden dazu Werte zwischen 3-95% angegeben (T. Zhou et al., PloS one, Vol. 8, Nr. 9, S. e74987, 2016; B. Setlow et al., J. of Bacteriology, 190(13), 4759, (2008)). Unter der ungünstigen Annahme von nur 3% DPA-Freisetzung kann man abschätzen, dass eine einzelne keimende Spore in einem Volumen von 1 nl die Konzentration auf 10mM erhöht. Das bedeutet, dass in einem Mikro-fluidic-Setting mit Dimensionen des Messbereiches im Bereich von 100 µm, daher der Einzelsporennachweis möglich sein sollte.

Die vorliegende Erfindung wird weiterhin unter Bezugnahme auf die Abbildung beschrieben, die aber lediglich der Verdeutlichung der erfindungsgemäßen Lehren dient und in keiner Weise den Umfang der vorliegenden Erfindung beschränken soll. Die Abbildung ist lediglich als schematisch anzusehen und nicht notwendigerweise maßstabsgetreu.

**Abbildung 1** zeigt ein System zum Testen einer Dekontamination gemäß einer beispielhaften Ausführungsform, das zum Durchführen eines Verfahrens gemäß einer beispielhaften Ausführungsform geeignet ist.

Das System 100 umfasst einen Filter 110, der auf seiner Oberfläche Sporen 112 aufweist. Das System 100 umfasst ferner eine Dekontaminationseinheit 150, in der der Filter 110 einer Dekontamination unterzogen wird. Stromaufwärts in der gezeigten Anordnung ist der Filter 110 fluidisch mit einer Wascheinheit 120, 122 verbunden, die einen Vorratsbehälter 120 für eine Waschflüssigkeit und eine Zuleitung 122 vom Vorratsbehälter 120 zum Filter 110 über eine Pumpvorrichtung 160 umfasst. Ebenfalls stromaufwärts ist der Filter 110 fluidisch mit einer Kontakt- und Inkubationseinheit 130, 132 verbunden, die einen Vorratsbehälter 130 für einen Keiminduktor und eine Zuleitung 132 vom Vorratsbehälter 130 zum Filter 110 über die Pumpvorrichtung 160 umfasst. In der gezeigten Anordnung wird nach dem Keimen der noch lebensfähigen Sporen die freigesetzte DPA stromabwärts des Filters 110 mittels einer Zugabevorrichtung 142 über ein Ventil 146 mit einer Lösung von Lanthanoid-Ionen in Kontakt gebracht und vermischt und der dabei erhaltene Lanthanoid-DPA-Komplex wird mittels eines Fluoreszenzdetektors 144 erfasst. Die Zugabevorrichtung 142, der Fluoreszenzdetektor 144, das Ventil 146 sowie die dazwischenliegenden Leitungen stellen die Bestimmungseinheit 140 dar.

Die vorliegende Erfindung wurde an Hand spezifischer Ausführungsformen und Beispiele beschrieben. Die Erfindung ist aber nicht hierauf beschränkt und verschiedene Modifikationen hiervon sind möglich, ohne den Umfang der vorliegenden Erfindung zu verlassen.

## Patentansprüche

1. Verfahren zum Testen einer Dekontamination, wobei das Verfahren Folgendes umfasst:
Bereitstellen mindestens eines Filters, der Sporen aufweist;
Unterziehen des Filters einer Dekontamination;
Waschen des Filters;
Inkontaktbringen des Filters mit einem Keiminduktor und Inkubieren des Filters;
Bestimmen der von den Sporen bei der Keimung freigesetzten Dipicolinsäure.

2. Verfahren nach Anspruch 1,
wobei das Bereitstellen des Filters ein Ablagern von Sporen auf dem Filter umfasst; und/oder
wobei der Filter mindestens eines der folgenden Merkmale aufweist:
- der Filter ist aus einem Material gemacht, das aus der Gruppe, bestehend aus Polytetrafluorethylen (PTFE), Polyvinylidenfluorid (PVDF), Glasfaser und Nylon ausgewählt ist;
- der Filter hat eine Porengröße zwischen 0,1 und 1 µm.

3. Verfahren nach Anspruch 1 oder 2, wobei das Verfahren ferner ein Waschen des Filters vor dem Unterziehen des Filters einer Dekontamination umfasst.

4. Verfahren nach einem der Ansprüche 1 bis 3, wobei das Unterziehen des Filters einer Dekontamination einen Dekontaminationsvorgang umfasst, der aus der Gruppe, bestehend aus einer Heiß-Dampf-Sterilisation, einem Dekontaminationsprozess mit H₂O₂ und einem Reinigungs-Entkeimungsprozess in der Abwassertechnik, ausgewählt ist.

5. Verfahren nach einem der Ansprüche 1 bis 4, wobei das Waschen des Filters ein Durchleiten einer Waschlösung, insbesondere einer Pufferlösung, durch den Filter umfasst.

6. Verfahren nach einem der Ansprüche 1 bis 5, wobei das Verfahren nach dem Waschen des Filters ferner ein Erwärmen des Filters und/oder einer Waschflüssigkeit und ein anschließendes weiteres Waschen des Filters mit der Waschflüssigkeit umfasst.

7. Verfahren nach einem der Ansprüche 1 bis 6, wobei das Inkontaktbringen des Filters mit einem Keiminduktor eine Zugabe des Keiminduktors zu dem Filter für eine bestimmte Zeit umfasst.

8. Verfahren nach einem der Ansprüche 1 bis 7,
wobei der Keiminduktor ausgewählt ist aus der Gruppe, bestehend aus Valin, insbesondere L-Valin, L-Alanin oder eine Mischung aus L-Asparagin, D-Glucose, D-Fructose und Kalium-Ionen (AGFK), und/oder
wobei eine Lösung, die den Keiminduktor enthält, ferner eine Puffersubstanz enthält.

9. Verfahren nach einem der Ansprüche 1 bis 8, wobei das Bestimmen der von den Sporen bei der Keimung freigesetzten Dipicolinsäure ein Inkontaktbringen der bei der Keimung freigesetzten Dipicolinsäure mit Lanthanoid-Ionen und ein anschließendes Detektieren der Fluoreszenz des gebildeten Lanthanoid-Dipicolinsäure-Komplexes umfasst.

10. System (100) zum Testen einer Dekontamination, insbesondere zum Durchführen eines Verfahrens gemäß einem der Ansprüche 1 bis 9, wobei das System (100) Folgendes aufweist:
mindestens einen Filter (110), der Sporen (112) aufweist;
eine Dekontaminationseinheit (150);
eine Einheit (120, 122), die konfiguriert ist, den Filter (110) zu waschen;
eine Einheit (130, 132), die konfiguriert ist, den Filter (110) mit einem Keiminduktor in Kontakt zu bringen und zu inkubieren;
eine Einheit (140), die konfiguriert ist, die von den Sporen bei der Keimung freigesetzte Dipicolinsäure zu bestimmen.

11. System (100) nach Anspruch 10, wobei zumindest der Filter (110), die Einheit (120, 122), die konfiguriert ist, den Filter (110) zu waschen, die Einheit (130, 132), die konfiguriert ist, den Filter (110) mit einem Keiminduktor in Kontakt zu bringen und zu inkubieren und die Einheit (140), die konfiguriert ist, die von den Sporen bei der Keimung freigesetzte Dipicolinsäure zu bestimmen, fluidisch miteinander verbindbar sind.

12. System (100) nach Anspruch 10 oder 11, wobei die Einheit (140), die konfiguriert ist, die von den Sporen bei der Keimung freigesetzte Dipicolinsäure zu bestimmen, einen Fluoreszenzdetektor (144) umfasst.

13. System (100) nach einem der Ansprüche 10 bis 12, wobei das System ferner mindestens eine der folgenden Komponenten umfasst:
eine Pumpvorrichtung (160);
ein Ventil (146);
eine Zugabevorrichtung (142);
einen Vorratsbehälter (120, 130).

14. System (100) nach einem der Ansprüche 10 bis 13, wobei das System eine automatisiertes System (100) ist, das einen oder mehrere Vorratsbehälter (120, 130), eine oder mehrere Pumpvorrichtungen (160), einen Filterhalter, eine Einheit (140), die konfiguriert ist, die von den Sporen bei der Keimung freigesetzte Dipicolinsäure zu bestimmen, ein Datenerfassungs- und Kontrollsystem, eine Software einschließlich einer Benutzeroberfläche umfasst.

15. Verwendung eines Verfahrens nach einem der Ansprüche 1 bis 9 oder eines Systems nach einem der Ansprüche 10 bis 14 zum Bestimmen der Wirksamkeit einer Dekontamination.
